# EUROPEAN PATENT APPLICATION

(11) **EP 3 957 330 A1**
(43) Date of publication of application: **23.02.2022**
(21) Application number: 20217652.5
(22) Date of filing: 29.12.2020
(51) Int. Cl.: A61K 47/00

(54) **MULTIPLE CONCENTRATE FORMULATION FOR MEDICAL PRODUCTS**

(30) Priority: 22.08.2020 US 202063103771 P; 13.10.2020 US 202063204576 P
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

Two or more concentrates for preparation of a medical device, medical product, or first aid gel. The concentrates of the present disclosure are configured to be added, to be sold, transported, and stored in concentrated form to ease transportation, decrease the cost of transportation and storage, optimize storage space, minimize product cost, ease use of product, militate against risk of microbial contamination, and increase viscosity. A unique preservative gel has been discovered during the new process of heating monolaurin and sorbic acid in propylene glycol before cooling to form an antimicrobial gel. This is used in one of the concentrates as a preservative.

## Description

### CROSS-REFERENCE TO RELATED APPLICATIONS

This application claims the benefit of U.S. Provisional Application Serial No. 63/103,771, filed on August 22, 2020. The entire disclosure of the above application is hereby incorporated herein by reference.

This application claims the benefit of U.S. Provisional Application Serial No. 63/204,576 filed on October 13, 2020. The entire disclosure of the above application is hereby incorporated herein by reference.

### FIELD

The present disclosure relates to multiple concentrates and, more particularly, to a combination of concentrates for preparation of medical products such as a natural burn gel for full-thickness burns. One of the concentrates is a gelling agent and one of the concentrates is a unique new invention of a preservative gel containing monolaurin. The concentrates are sold as a product and after purchase are mixed with purified water in a one gallon jug to form a burn gel.

### BACKGROUND

Burns can cause significant morbidity and mortality. There is a significant risk of infection associated with burns, especially *Pseudomonas aeruginosa.* Burns can cause immunosuppression, therefore burn gels have to have antimicrobial efficacy against a broad spectrum of microbes including bacteria and fungi. Burn gels have to be thick enough to adhere to the wound site, but not so adherent to cause pain when the burn is cleaned. They have to cause minimal sensitivity as burn sites can have exposed, inflamed tissues that are likely to be sensitive to chemicals.

Early death by burns is often caused by dehydration leading to shock due to loss of fluids. Burns gels need to be able to inhibit dehydration to prevent fluid volume loss of patients.

There are several problems with current burn creams. Burn creams can be adherent to the point of causing significant pain when cleaned. They can also cause sensitization of the burn site causing allergic reactions or immune response.

Burns gels are advantageous for burns compared to burn creams because the main ingredient is water and wet wounds heal up to 3 times faster than dry wounds. The problem with burn gels is that they may have difficulties adhering to the burn site or may lack the viscosity to stick to the wound. Therefore burn gels have to be thick and adherent enough to maintain contact with the burn site. They may require a more viscous and adherent gel.

There are times that a higher viscosity gel is desired to increased ultrasound conduction, increase electrical conduction, or to provide more adhesion. The inventor has discovered that he can create a more viscous product by admixing two or more separate concentrates with water. In one non-limiting example, it was discovered that by admixing water with two separate concentrates, one a gelling concentrate and another preservative concentrate, created a gel with a higher viscosity.

Burns may require a considerable amount of gel to cover a large burn surface area. This can cause quite an expense to cover a large burn surface area. Burn gels can take up significant storage space in a hospital or storage facilities and can be expensive to ship.

There is a continuing need for concentrates for preparation of a medical device, medical product, or first aid product, which is configured to be mixed on site. This saves storage space and shipping costs to ship and store concentrates instead of a product that consists mostly of water. Desirably, the concentrates are resistant to microbial growth and the final product has a high viscosity. Concentrates have a lower water activity level, or amount of water available for microbes to use which minimizes the likelihood of microbial contamination prior to use and increases safety of the product.

The present invention relates a discovery that has been made that mixing monolaurin and sorbic acid with propylene glycol and heating creates a gel upon cooling. Monolaurin is a natural antimicrobial that inhibits microbes by damaging the cell membrane or viral membrane at an acidic pH. Monolaurin in particular has the potential to be a broad spectrum preservative. It has been found to kill or inhibit enveloped viruses, bacteria, and fungi, which makes it a broad spectrum preservative and antimicrobial. Sorbic acid is a chemical found in nature that is used as a preservative. Propylene glycol is a solvent and humectant used as an antifreeze that has antimicrobial properties at high concentrations. Tables 1 and 2 show the minimal inhibitory concentration of monolaurin against *Pseudomonas Aeruginosa* and *Staphylococcus epidermidis.* Table 3 shows the minimal inhibitory concentration of sorbic acid against *Escherichia coli* and *Staphylococcus Aureus.*

The problem with monolaurin in the past was that it has limited water solubility. The inventor discovered that also by heating monolaurin with a water solution, such as aloe vera juice, ginger juice, or honey at a ratio of 1:3 to 1:10, creates a gel. Another discovery was that after heating monolaurin with a propylene glycol or phenoxyethanol, at a 1:3 to a 1:10 ratio created a gel at room temperature that melts at body temperature. This is advantageous when one wants an antimicrobial effect directly on the skin after water has evaporated.

These gels can be admixed with a water-based gel to form the final product. The advantage with monolaurin is that it can be used as a natural antimicrobial and when heated and mixed with a solvent, used as a topical preservative or antimicrobial.

Monolaurin has a synergistic antimicrobial effect with chelating agents. Chelating agents are chemicals that bind to metal ions, preventing microbes from using them. Table 3 Shows this synergistic effect of monolaurin with EDTA.

A variety of natural preservatives can be added before heating, such as curcumin, sorbic acid, vanillin, and essential oils. The added products at low concentration do not impact the gel formation, but are able to provide a synergistic effect, creating a synergistic natural antimicrobial or preservative gel. This natural gel can be admixed with a water based gel to create the final product.

### SUMMARY

In concordance with the instant disclosure, a combination of concentrates for preparation of a medical device, medical product, or first aid product, which is configured to be mixed onsite, have an increased viscosity, use natural ingredients, and are resistant to microbial growth has surprisingly been discovered.

The present application relates to a gel adapted to be manufactured, transported, and sold in multiple concentrates that can only be used after water or an alcohol is added.

In one embodiment, two concentrates for preparation of a burn gel, includes a concentrate containing a gelling agent and a humectant and another concentrate containing a preservative and a humectant. This provides a benefit of higher viscosity when the gelling agent is mixed with water separately before the preservative is added.

In one embodiment, the preservative concentrate contains monolaurin, a combination of lauric acid and glycerine.

In a further embodiment, a method of preparing a hand sanitizer includes a step of admixing water and/or alcohol and two concentrates for preparation of a thick, moisturizing, hand sanitizer. Alcohol has a drying effect on the skin. A thick moisturizing antimicrobial gel that adheres to the skin provides a thin layer over the skin that protects it from dehydration. This is advantageous over a thinner hand sanitizer that may dry out the skin. When the skin dries, it cracks, which increases the risk of eczema, allergic reactions, and infection.

The concentrates of the present disclosure is configured to be sold, transported, and stored in a concentrated form to ease transportation. In addition, the concentrates of the present disclosure will decrease the cost of transportation and storage; optimize storage space; be cost-effective; and minimize risk of product contamination.

There are several benefits of a concentrated medical device, medical product, or first aid product. The first is easier and more cost-effective shipping. A certain percentage of medical devices, medical products, or first aid products are water that increases weight and space during shipping. Shipping size and weight may also be smaller as the packaging for shipping could also be much smaller.

A second benefit of concentrated medical devices, medical products, or first aid products is that they takes up less space than regular medical devices, medical products, or first aid products in storage. This benefits health care facilities by decreasing the amount of required storage space.

Ease of accessibility and cost is also a benefit. Instead of having to try to pour viscous medical devices, medical products, or first aid products out of a large container into a smaller container for use, one just has to add water to two or more medical device, medical product, or first aid product concentrates in a bottle and mix to create the final product.

The concentrates have a lower water activity level, or amount of water available for microbes to use. This minimizes the likelihood of microbial contamination prior to use and increases safety of the product. Bacteria and candida cannot multiply in a product with a water activity level less than 0.87.

By using a combination of concentrates and mixing each separately with water, the medical device, medical product, or first aid product can reach a higher viscosity by mixing the gelling agent with water first before adding the preservative concentrate.

In most particular embodiments, one concentrate for medical devices contains a gelling agent and one concentrate contains a preservative. The gel concentrates can be admixed with other ingredients to create the final product or can be sold as the final product. The final product has a water activity level of less than 0.87 and requires the addition of water prior to use. The concentrates may be used in at least one of an burn gel, ultrasound conducting product, a sterile ultrasound gel, provided as a solution or gel, provided as a disintegrating packet, provided in an all-natural product as defined by FDA guidelines, an electrically conductive gel, and a disinfectant, as non-limiting examples.

In yet another particular embodiment, concentrates for medical supplies or first aid supplies. The concentrates can be admixed with other ingredients to create the final product or can be sold as the final product. The concentrates have a water activity level of less than 0.87 and requires the addition of water prior to use. The concentrates may be used in a water soluble lubricant, a sterile water soluble lubricant, a sanitizer, a wound gel, a product containing aloe vera powder, an ulcer gel, a massage gel, a sunscreen, and an insect repellant, as non-limiting examples.

In yet a further particular embodiment, concentrates for alcohol sanitizers can be mixed with alcohol. The concentrates can be admixed with other ingredients to create the final product or can be sold as the final product. The concentrates would have a water activity level of less than 0.87 and would require the addition of an alcohol prior to use.

### DETAILED DESCRIPTION

The following description is merely exemplary in nature and is not intended to limit the present disclosure, application, or uses. In respect of the methods disclosed, the order of the steps presented is exemplary in nature, and thus, is not necessary or critical unless otherwise disclosed.

The terminology used in the specification provided herein below is hereby defined to include similar and/or equivalent terms, and/or alternative embodiments that would be considered obvious to one skilled in the art given the teachings of the present patent application. Additionally, the words "a," "an," and "one" are defined to include one or more of the referenced item unless specifically stated otherwise.

The term "ultrasound gel" refers to scanning gel or gels, transmission gel or gels, ultrasound gel or gels, ultrasound lotion or lotions, or any other solution designed or sold for the purpose of transmitting ultrasound waves.

The term "burn gel" refers to gels designed or used for the purpose of providing pain relief and / or healing third degree or full-thickness burns.

The term "preservative" refers to a substance used to militate against decay.

The use of percentages in describing particular formulations hereinbelow should be understood as referring to percentage by weight relative to a total weight of the formulation, unless otherwise disclosed.

The present disclosure is directed to concentrates for the preparation of a burn gel. The burn gel may include a gelling concentrate and a preservative concentrate.

The gelling concentrate may be configured to provide a viscosity that is suitable for use as burn gel. Also, the preservative concentrate may provide low irritancy and non-sensitizing properties. In addition, the preservative concentrate may have properties such that the preservative concentrate is not bio-absorbed or metabolized where utilized in the burn gel. The gelling concentrate may further be dispersed in water without pre-dispersion. In some embodiments, the gelling concentrate may have a moisture level less than about 10%.

The gelling concentrate may have a pH in water which is suitable for use on human wounds. In certain embodiments, the gelling concentrate may be "pre-neutralized," such that no additional ingredients are required to adjust the pH of the gelling agent. In further embodiments, the gelling concentrate may include a basic additive, which may change the overall pH of the ultrasound gel in order to reach the suitable pH range disclosed herein.

In a non-limiting example, the gelling concentrate may contain hydroxypropyl methylcellulose. Hydroxypropyl methylcellulose may be present in the concentrate of about 7.5 grams. The concentration of hydroxypropyl methylcellulose in the burn gel may be about 1%. A skilled artisan may select other suitable amounts of hydroxypropyl methylcellulose for both the concentrate and the final burn gel, as desired.

Although the above-mentioned gelling agent has been found to be useful, one of ordinary skill in the art may also employ other suitable gelling agents within the scope of the disclosure.

In further non-limiting embodiments, the concentrate may include natural gelling ingredients. In these embodiments, the gelling agent may be vegetable glycerin, agar, alginates, processed cellulose, gelatin, gum arabic, gum tragacanth, konjac gum, monolaurin, xanthan gum, or a combination thereof. A skilled artisan may select other suitable gelling agents, as desired.

Water activity may be defined as the ratio of the vapor pressure of water in a substance to the vapor pressure of pure water at the same temperature. Microorganisms require a minimum level of water activity in order to grow and, thus, water activity reduction may be used to control microbial growth.

Accordingly, it is desirable for the concentrate to have a low water activity level. For example, the water activity level of the concentrate may be less than about 0.87. The at least one humectant may be used to reduce the water activity level of the concentrate. The humectant may bind to the free water molecules in the concentrate and thereby reduce the water activity level. As non-limiting examples, the humectant may be butylene glycol, caprylyl glycol, dextrose, fructose, glycerol, glycine, vegetable glycerine, glucose, hexylene glycol, honey, malic acid, nitric acid, propylene glycol, salt, sorbitol, sucrose, and tartaric acid.

In one non-limiting example, the humectant may be glycerol. Advantageously, glycerol may militate against undesirable clumping within the concentrate, and therefore, improve the dispersibility of the concentrate. A skilled artisan may select other suitable humectants and amounts of humectant in the concentrate, as desired.

The at least one preservative may be non-irritating and non-sensitizing. The preservative may be non-reactive such that the preservative does not undesirably react with other components of the concentrate, the air, or light. At least one preservative may be effective against gram-negative and gram-positive bacteria, and the yeast Candida albicans, for example. Desirably, the preservative may not release formaldehyde and may be paraben free. At least one preservative may also be a combination of multiple preservatives. As non-limiting examples, the preservative may be a combination of sorbic acid, phenoxyethanol, and monolaurin. A skilled artisan may select other suitable preservatives and amounts of humectant in the concentrate, as desired.

In further non-limiting embodiments, the concentrates may include other natural preservatives or antimicrobials. In these embodiments, the preservative agent may be an essential oil, a catechin, or flavonoid or a combination thereof. A skilled artisan may select other suitable natural antimicrobials, as desired.

In certain embodiments, the concentrate may include at least one chelating agent. The chelating agent may be one or more chemicals used to bind metal ions. Advantageously, the chelating agent may militate against microbes. The chelating agent may be selected from a list of approved chelating ingredients including disodium EDTA (ethylenediaminetetraacetic acid), tetrasodium EDTA (ethylenediaminetetraacetic acid), citric acid, sodium citrate, Sodium Phytate, Phytic Acid, sodium gluconate, EDDS (trisodium ethylenediamine disuccinate), Cyclodextrin, and activated carbon. This list does not limit possible chelating agents and a skilled artisan may select other suitable chelating agents.

In a most particular non-limiting example, a burn gel concentrate may be a gelling concentrate comprising a solution of about 3% vegetable glycerin, about 0.4% xanthan gum, about 1% konjac gum, about 1% sodium hydroxymethyl cellulose, and about 0.2% disodium ethylenediaminetetraacetic acid (EDTA). The second burn gel concentrate may be a preservative concentrate comprising a gel of about 0.5% phenoxyethanol, about 0.04 % star anise essential oil, about 0.1% sorbic acid, about 0.1% vanillin, about 0.5% monolaurin, and about 2% propylene glycol. The percentages being weight percentages based on a final gel product after water is added.

In this non-limiting example, the gelling concentrate is placed in a clean one gallon bottle. About 12 cups or about 2,839 milliliters of purified water is added to the bottle at a set filling line and admixed through shaking for 30 seconds. The preservative concentrate is added and admixed through shaking a second time for another 30 seconds. Within 30 minutes the burn gel thickens and is available for use. A hand pump may be screwed onto the bottle and the burn gel may then be applied to full-thickness burns.

Please note that xanthan gum in combination with konjac gum has a synergistic gelling effect. Sodium hydroxymethyl cellulose in combination with xanthan gum has a synergistic higher viscosity. Sodium hydroxymethyl cellulose in combination with knojac root has a synergistic higher viscosity. Monolaurin with EDTA have a synergistic antibacterial effect. Monolaurin with sorbic acid have a synergistic antiviral effect against enveloped viruses.

The gel may melt at body temperature, advantageously allowing the gel to adhere to the application site and form a protective barrier to maintain moisture of the wound site after application.

It should be appreciated that the concentrates may be provided in a plurality of forms. In one embodiment, the concentrates may be provided as concentrated solutions configured to be admixed and thereby diluted with ethanol to form a sanitizer gel. In other embodiments the concentrates may be gels configured to be admixed with honey to form a wound gel. In a third embodiment one concentrate is a solution and one concentrate is a gel that are configured to be admixed with purified water to form the burn gel.

As disclosed herein, the concentrates may be provided in various final packaging. A bottle may be used to dispense a predetermined amount of liquid concentrates, as needed.

Where the concentrates are in a powdered form, the powder may be provided in bulk. A user may measure a predetermine amount of the powders, as needed. Alternatively, the powders may be pressed into tablets. The tablet may contain the predetermined amount of powder, such that a user does not need to measure the powder. A skilled artisan may select other suitable methods and materials to form powders or tablets, as desired.

In other embodiments, the concentrates may be provided in dissolvable packets. The dissolvable packets may contain the concentrates in gel form, solution form, powder form, or in liquid form. The user may select each of two dissolvable packets, as needed, and place the packets in water, where the packets will dissolve, and the concentrate may admix with purified water to form the burn gel. As one non-limiting example, the dissolvable packet may be thermoformed. Sheets of water-soluble film may be sealed together to encapsulate the concentrates separately. For example, the sheets may be fabricated from polyvinyl alcohol, which is a water-soluble synthetic polymer. The sheets may be heat sealed, as a non-limiting example. A skilled artisan may select other suitable methods and materials to encapsulate the concentrates, as desired.

The present disclosure further contemplates a method of preparing the burn gel. The method includes a first step of providing the concentrates. As disclosed hereinabove, the concentrates may be provided in a plurality of forms and packaging.

The method may then include a second step of admixing water and the concentrates. More particularly, where the user requires the burn gel, the user may transfer the predetermined amount of the concentrates, as a non-limiting example, a gel packet and a solution packet, to a bottle configured for the final use. The user may then place a predetermined amount of purified water to the bottle. The user may then mix the concentrates and water until the burn gel is formed. Optionally, the user may adjust the viscosity by adding more water, as desired.

While the concentrates have been described for use in forming the burn gel, it should be appreciated that the present disclosure contemplates a variety of final uses for the gel formed from the concentrate where admixed with water. As non-limiting examples, end uses for the gel may include lubricants, sanitizers, wound gels, ultrasound gels, ulcer gels, massage gels, sunscreens, and insect repellants. A skilled artisan may utilize the concentrates in suitable uses, which require a gel.

It should be appreciated that further ingredients may be admixed into the final gel, as needed, based on the final use. As one non-limiting example, alcohol may be admixed with the concentrates and water to form a sanitizing gel.

In yet another embodiment of the disclosure, an alcohol such as ethanol may be used instead of water to provide sanitization effect, for example, where the concentrate is adapted for use as a hand sanitizer.

Other ingredients can be included in the present compositions, such as various excipients, including one or more antiadherents (e.g., magnesium stearate), binders (e.g., saccharides, gelatin, polymers), disintegrants (e.g., polyvinylpyrrolidone, carboxymethyl cellulose, modified starches), scents, and colors. A skilled artisan may select suitable excipients in order to facilitate both the concentration of the ingredients into a container, dissolvable packet, or tablet, and the later preparation of the final gel products, as desired.

As may be presented herein, the language "consisting essentially of" is meant to limit the scope of the claim to the specified materials that do not materially affect the basic and novel characteristics of the additive. It should be appreciated that ingredients that materially affect the additive may adversely affect the water activity level or the gelling properties of the concentrate. Thus, the concentrate consisting essentially of certain ingredients described hereinabove, excludes ingredients that may improve or adversely affect the water activity level or the gelling properties of the concentrate, which would materially affect the basic and novel characteristics of the concentrate.

Tables 1-3 demonstrate antibacterial effects of monolaurin. Table 3 demonstrates the antibacterial properties of sorbic acid and synergy of monolaurin with EDTA.

**Table 1. Minimal Inhibitory Concentration in micrograms per milliliter of monolaurin and nanoparticle monolaurin.**

| | Monolaurin | Nanoparticle monolaurin |
|---|---|---|
| *Pseudomonas Aeruginosa* | 62.5 | 15.62 |

**Table 2. Minimal Inhibitory Concentration in micrograms per milliliter of monolaurin and rifampicin at 24 hours.**

| | Monolaurin | Rifampicin |
|---|---|---|
| *Staphylococcus epidermidis* | 1953 | 488 |

**Table 3. Minimal Inhibitory Concentration in micrograms per milliliter of monolaurin, sorbic acid, and monolaurin with EDTA at 24 hours.**

| | pH | Monolaurin | Sorbic Acid | Monolaurin + EDTA |
|---|---|---|---|---|
| *Escherichia coli* | 7 | >4,000 | >5,000 | 2,000 |
| | 6 | 2,000 | 1,250 | |
| | 5 | 500 | 312.5 | |
| | | | | |
| *Staphylococcus Aureus* | 7 | 128 | 1,250 | 32 |
| | 6 | 64 | 312.5 | |
| | 5 | 16 | 156.25 | |

While certain representative embodiments and details have been shown for purposes of illustrating the invention, it will be apparent to those skilled in the art that various changes may be made without departing from the scope of the disclosure, which is further described in the following appended claims.

## Claims

1. A medical device, medical product, or first aid product, comprising:
at least two concentrates that are sold and transported as a concentrate and admixed with at least one of water and an alcohol after transport and prior to use.

2. A concentrate of Claim 1, wherein the concentrate is a concentrated solution.

3. A concentrate of Claim 2, wherein the concentrated solution contains two or more gelling agents.

4. A concentrate of Claim 2, wherein the concentrate solution contains a chelating agent.

5. A concentrate of Claim 1, wherein the concentrate is a gel.

6. A concentrate of Claim 5, wherein the concentrate comprises at least one monoglyceride.

7. A concentrate of Claim 6, wherein the concentrate comprises monolaurin at a concentration of 0.5% of the final product.

8. Concentrates of Claim 1, wherein the concentrates have a water activity level of less than about 0.87.

9. A preservative gel, antimicrobial gel, or gelling agent comprising:
at least one of propylene glycol, butylene glycol, caprylyl glycol, hexylene glycol, and phenoxyethanol;
at least one of monolaurin, monomyristin, monocaprin and monocaprylin;
and at least one of a preservative and an antimicrobial.

10. A gelling agent in Claim #9 that is used as a preservative.

11. A gelling agent in Claim #9 that is used as an antimicrobial.

12. The process of:
melting at least one of curcumin, monoglyceride, sorbic acid, and vanillin;
with or without admixing at least one of a lipid soluble preservative and a lipid soluble antimicrobial;
admixing with at least one of water, propylene glycol, butylene glycol, caprylyl glycol, hexylene glycol, and phenoxyethanol;
and cooling to form a gel.

13. The process in claim #12 of melting monolaurin and sorbic acid; and admixing with phenoxyethanol and propylene glycol.

14. The process in claim #12 of melting monolaurin, sorbic acid, and vanillin; and admixing with phenoxyethanol and propylene glycol.

15. The process in claim #12 of melting monolaurin, sorbic acid, vanillin; admixing with star anise essential oil; and admixing with phenoxyethanol and propylene glycol.
